(19) Europäisches Patentamt
European Patent Office
Office européen des brevets

(11) Veröffentlichungsnummer: **0 020 955**
**A1**

(12) **EUROPÄISCHE PATENTANMELDUNG**

(21) Anmeldenummer: **80102452.2**

(51) Int. Cl.³: **C 07 D 249/08**, A 61 K 31/41

(22) Anmeldetag: **06.05.80**

(30) Priorität: **19.05.79 DE 2920437**

(43) Veröffentlichungstag der Anmeldung: **07.01.81**
**Patentblatt 81/1**

(84) Benannte Vertragsstaaten: **AT BE CH DE FR GB IT LI NL SE**

(71) Anmelder: **BAYER AG, Zentralbereich Patente, Marken und Lizenzen, D-5090 Leverkusen 1, Bayerwerk (DE)**

(72) Erfinder: **Regel, Erik, Ing.-grad., Bergerheide 72a, D-5600 Wuppertal 1 (DE)**
Erfinder: **Draber, Wilfried, Dr., In den Birken 81, D-5600 Wuppertal 1 (DE)**
Erfinder: **Büchel, Karl Heinz, Dr. Prof., Bergerheide 62, D-5600 Wuppertal 1 (DE)**
Erfinder: **Plempel, Manfred, Dr., Pahlkestrasse 5, D-5600 Wuppertal 1 (DE)**
Erfinder: **Haller, Ingo, Dr., Viktoriastrasse 99, D-5600 Wuppertal 1 (DE)**

(54) **Geometrische Isomere von 4,4-Dimethyl-1-phenyl-2-(1,2,4-triazol-1-yl)-1-penten-3-olen, Verfahren zu ihrer Herstellung, sie enthaltendes Arzneimittel, Verfahren zur Herstellung von antimykotischen Mitteln und zur Behandlung von Mykosen.**

(57) Die geometrischen Formen B von 4,4-Dimethyl-1-phenyl-2-(1,2,4-triazol-1-yl)-1-penten-3-olen der allgemeinen Formel

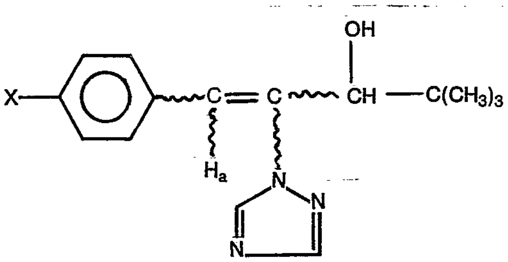

(I)

in welcher

X für Halogen, Cyano oder gegebenenfalls substituiertes Phenyl steht,

und deren physiologisch verträglichen Säureadditions-Salze weisen starke antimykotische Eigenschaften auf.

EP 0 020 955 A1

BAYER AKTIENGESELLSCHAFT 5090 Leverkusen, Bayerwerk
Zentralbereich
Patente, Marken und Lizenzen Si-kl
Ia(PHa)

**BEZEICHNUNG GEÄNDERT**
**siehe Titelseite**

Geometrische Isomere von 4,4-Dimethyl-1-phenyl-2-(1,2,4-triazol-1-yl)-1-penten-3-olen, Verfahren zu ihrer Herstellung sowie ihre Verwendung als Arzneimittel

Die vorliegende Erfindung betrifft neue geometrische Isomere von 4,4-Dimethyl-1-phenyl-2-(1,2,4-triazol-1-yl)-1-penten-3-olen, ein Verfahren zu ihrer Herstellung sowie ihre Verwendung als Arzneimittel, insbesondere als Antimykotika.

Es ist bereits bekannt geworden, daß geometrische Isomerengemische von 4,4-Dimethyl-1-phenyl-2-(1,2,4-triazol-1-yl)-1-penten-3-olen allgemein antimykotische Wirkung aufweisen (vergleiche DE-OS 28 38 847).

Es wurden nun als neue Verbindungen die geometrischen Formen B (siehe nachfolgende Erläuterung) von 4,4-Dimethyl-1-phenyl-2-(1,2,4-triazol-1-yl)-1-penten-3-olen der allgemeinen Formel

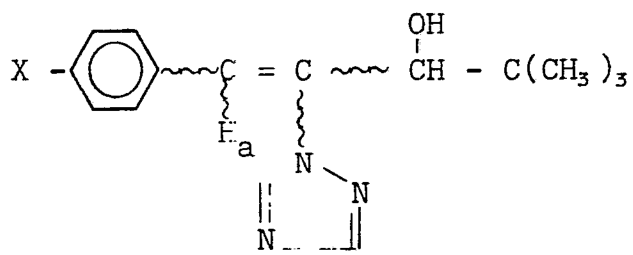

(I)

in welcher

X für Halogen, Cyano oder gegebenenfalls substituiertes Phenyl steht,

und deren physiologisch verträglichen Säureadditions-Salze gefunden. Sie weisen starke antimykotische Eigenschaften auf.

Zur Erläuterung sei hierzu vermerkt:
Geometrische Isomeren an einer Doppelbindung werden durch die Präfixe cis (Z, abgeleitet von zusammen) und trans (E, abgeleitet von entgegen) unterschieden. Bei den erfindungsgemäßen Verbindungen ist die Zuordnung aufgrund der noch nicht bestimmten absoluten Konfiguration nur bedingt möglich; dies wird durch die Wellenlinien in der Formel (I) zum Ausdruck gebracht. Entsprechend wird hier in Form A und Form B unterteilt, die sich nach ihren physikalisch-chemischen Eigenschaften eindeutig charakterisieren lassen.

Ein eindeutiges Charakterisierungsmerkmal ist die $H^1$-Kernresonanz des Protons $H_a$ in den entsprechenden Ausgangsketonen der Formel (II). Diese erfogt bei den B-Formen gegenüber den A-Formen bei höherem Feld.

Die Verbindungen der Formel (I) besitzen ein asymmetrisches Kohlenstoffatom, so daß sie in zwei optischen Isomerenformen anfallen. Die vorliegende Erfindung betrifft sowohl die einzelnen optischen Isomeren als auch die Isomeren-Gemische.

Weiterhin wurde gefunden, daß man die geometrischen Formen B der Verbindungen der Formel (I) erhält, wenn man die geometrischen Formen B von 4,4-Dimethyl-1-phenyl-2-(1,2,4-triazol-1-yl)-1-penten-3-onen der Formel

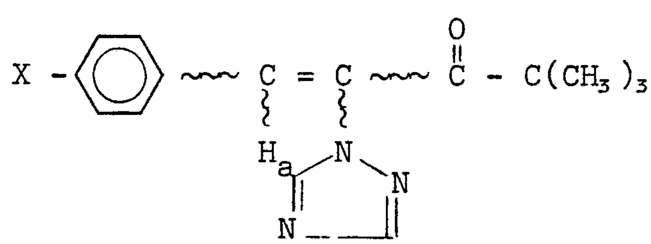
(II)

in welcher

X die oben angegebene Bedeutung hat,

mit komplexen Hydriden, gegebenenfalls in Gegenwart eines Verdünnungsmittels; oder mit Aluminium-isopropylat in Gegenwart eines Verdünnungsmittels reduziert.

Weiterhin können die erfindungsgemäß erhältlichen Formen B von 4,4-Dimethyl-1-phenyl-2-(1,2,4-triazol-1-yl)-1-penten-3-olen der Formel (I) durch Umsetzen mit Säuren in die Salze überführt werden.

Uebrraschenderweise zeigen die erfindungsgemäßen Formen B von 4,4-Dimethyl-1-phenyl-2-(1,2,4-triazol-1-yl)-1-penten-3-olen der Formel (I) eine erheblich bessere, therapeutisch nutzbare Wirksamkeit als die aus dem Stand der Teshnik bekannten entsprechenden geometrischen Isomeren-Gemische. Die erfindungsgemäßen Stoffe stellen somit eine wertvolle Bereicherung der Pharmazie dar.

Aufgrund des bekannten Standes der Technik konnte keineswegs erwartet werden, daß sich die erfindungsgemäßen Formen B der Formel (I) durch sehr gute antimykotische Eigenschaften auszeichnen, während die analogen geometrischen Formen A dieser Verbindungen als Antimykotika nur schwach aktiv sind.

Die erfindungsgemäßen geometrischen Formen B von 4,4-Dimethyl-1-phenyl-2-(1,2,4-triazol-1-yl)-1-penten-3-olen sind durch die Formel (I) allgemein definiert. In dieser Formel steht X vorzugsweise für Fluor, Chlor, Cyano oder gegebenenfalls durch Chlor substituiertes Phenyl.

Besonders bevorzugt ist diejenige Verbindung, in der X für Chlor steht.

Verwendet man neben der geometrischen Form B von 1-(4-Chlorphenyl)-4,4-dimethyl-2-(1,2,4-triazol-1-yl)-1-penten-3-on Natriumborhydrid als Ausgangsstoffe, so kann der Reaktionsablauf durch das folgende Formelschema wiedergegeben werden:

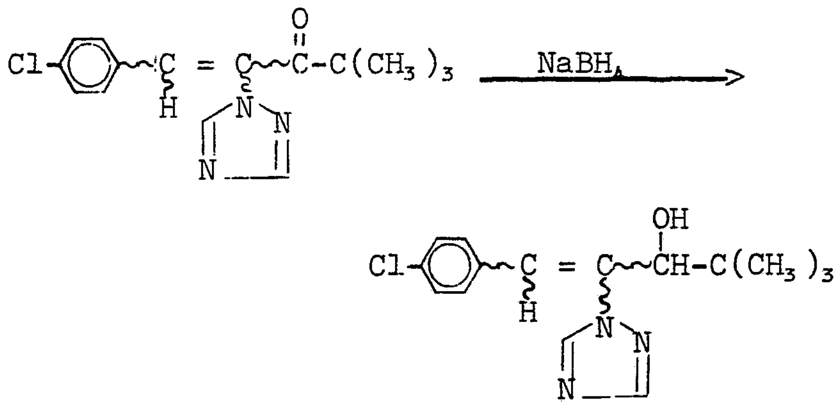


Die als Ausgangsstoffe zu verwendenden geometrischen Formen B von 4,4-Dimethyl-1-phenyl-2-(1,2,4-triazol-1-yl)-1-penten-3-onen der Formel (II) sind noch nicht bekannt. Sie können jedoch in allgemein bekannter Art und Weise erhalten werden (vergleiche auch die Angaben in der DE-OS 28 38 847), indem man 3,3-Dimethyl-1-(1,2,4-triazol-1-yl)-butan-2-on der Formel

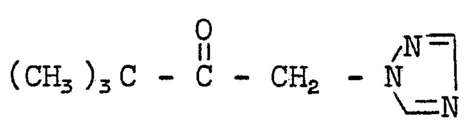 (III)

mit Aldehyden der Formel

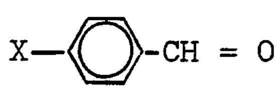 (IV)

in welcher

X die oben angegebene Bedeutung hat,

in Gegenwart eines inerten organischen Lösungsmittels, wie beispielsweise aromatische Kohlenwasserstoffe oder

Alkohole, und in Gegenwart eines Katalysators, wobei man alle üblicherweise verwendbaren sauren und insbesondere auch basischen Katalysatoren sowie deren Puffergemische einsetzen kann, wie beispielsweise Eisessig, Pyridin, Piperidin und insbesondere Piperidinacetat, bei Temperaturen zwischen 20 und 160°C umsetzt. Zur Isolierung der geometrischen Formen B der Formel (II) wird das Isomerengemisch (bestehend aus Form B und Form A) nach üblichen Methoden, wie z.B. aufgrund unterschiedlicher Löslichkeit, durch Salzbildung oder chromatographisch getrennt. Eine eindeutige Strukturzuordnung erfolgt aufgrund der NMR-Daten, insbesondere des Protons $H_a$.

Das 3,3-Dimethyl-1-(1,2,4-triazol-1-yl)-butan-2-on der Formel (III) ist bekannt (vergleiche DE-OS 24 31 407 und DE-OS 26 38 470).

Die Aldehyde der Formel (IV) sind allgemein bekannte Verbindungen der organischen Chemie.

Wird die erfindungsgemäße Reduktion mit komplexen Hydriden durchgeführt, so kommen als Verdünnungsmittel vorzugsweise polare organische Lösungsmittel infrage. Hierzu gehören vorzugsweise Alkohole, wie Methanol,Ethanol, Butanol, Isopropanol; sowie Ether, wie Diethylether oder Tetrahydrofuran. Die Reaktion wird im allgemeinen bei 0 bis 30°C, vorzugsweise bei Raumtemperatur, durchgeführt. Hierzu setzt man auf 1 Mol des Ketons der Formel (II) etwa 1 Mol eines komplexen Hydrids, wie Natriumborhydrid oder Lithiumalanat, ein. Die Isolierung der erfindungsgemäßen Stoffe erfolgt in üblicher Weise.

In einer besonderen Ausführungsform wird die Reduktion mit komplexen Hydriden in Gegenwart von komplexbildenden

Metallsalzen, wie z.B. Cer-(III)-chlorid, durchgeführt, was zu einer Verhinderung der sonst als Nebenreaktion ablaufenden Hydrierung der C=C-Doppelbindung führt (vergleiche Journal Chem.Soc.Chem.Communications 1978, 601 sowie die Herstellungsbeispiele).

Arbeitet man mit Aluminiumisopropylat als Reduktionsmittel, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden; im allgemeinen arbeitet man zwischen 20 und 120°C, vorzugsweise bei 50 bis 100°C. Zur Durchführung der Reaktion setzt man auf 1 Mol des Ketons der Formel (II) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von Säureadditionssalzen der Verbindungen der Formel (I) kommen alle physiologisch verträglichen Säuren infrage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure sowie Sulfonsäuren, wie z.B. p-Toluolsulfonsäure und 1,5-Naphthalindisulfonsäure.

Die Salze der Verbindungen der Formel (I) können in einfacher Weise nach üblichen Salzbildungsmethoden, z.B. durch Lösen der Verbindungen der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäß verwendbaren Verbindungen der Formel (I), ihre Säureadditions-Salze und Metallsalz-Komplexe weisen antimikrobielle, insbesondere starke antimykotische Wirkungen auf. Sie besitzen ein sehr breites antimykotisches Wirkungsspektrum, insbesondere gegen Dermatophyten und Sproßpilze sowie bisphasische Pilze, z.B. gegen Candida-Arten, wie Candida albicans, Epidermophyton-Arten, wie Epidermophyton floccosum, Aspergillus-Arten, wie Aspergillus niger und Aspergillus fumigatus, wie Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkungder bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:
Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen,

Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumbicarbonat, (e) Lösungsverzögerer, z.B. Paraffin und (f) Resorptionsbeschleuniger, z.B. quartenäre Ammoniumverbindungen, (g) Netzmittel, z.B. Cetylalkohol, Glycerinmonostearat, (h) Adsorptionsmittel, z.B. Kaolin und Bentonit und (i) Gleitmittel, z.B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z.B. Polyäthylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z.B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesaöl, Glycerin, Glycerinformal, Tetrahydrofurfurylalkohol, Polyäthylenglykole und Fettsäureester des Sorbitans oder Gemische dieser Stoffe enthalten.



BAD ORIGINAL

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Äthylalkohol, Propylenglykol, Suspendiermittel, z.B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z.B. Pfefferminzöl und Eukalyptusöl und Süßmittel, z.B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden , z.B. durch Mischen des oder der Wirkstoffe mit dem oder den Trägerstoffen.



BAD ORIGINAL

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Beispiel A

Antimykotische in-vitro-Wirksamkeit

Versuchsbeschreibung:

Die in-vitro-Wirksamkeit - gemessen anhand der minimalen Hemmkonzentration - wurde im Agarverdünnungstest ermittelt. Auf Serien von Kimmig - Agarplatten, die die entsprechenden Wirkstoffkonzentrationen im Agar gelöst enthielten, wurden mit Hilfe eines automatischen Beimpfungsgerätes Proben der Keimsuspesionen tropfenförmig aufgebracht. Das Inokulum betrug 2 - 5 x $10^3$ Pilzpartikel pro Impfpunkt. Im Falle der Hefen lag die Bebrütungsdauer bei 24 Stunden, im Falle der Dermatophyten bei 96 Stunden; Bebrütungstemperatur 27°C.

In diesen Versuchen zeigten die erfindungsgemäßen Formen B bessere Wirksamkeit als die bekannten geometrischen Isomerengemische.

Die Ergebnisse sind in Tabelle A zusammengefaßt.

Tabelle: A Antimykotische in-vitro-Wirksamkeit

| Wirkstoff | Minimale Hemmkonzentration in mcg/ml bei: Trichophyton mentagr. | Microsporon canis | Candida albicans | Torulopsis glabrata | Aspergillus fumigatus |
|---|---|---|---|---|---|
| 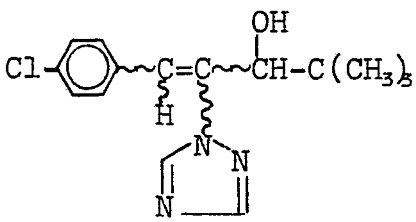 geometrisches Isomerengemisch (bekannt) | <1 | 8 | 8 | 4 | 8 |
| Form A | 4 | 32 | 4 | 1 | 32 |
| Form B(1) | <1 | <1 | <1 | <1 | 1-4 |

Beispiel B

Antimikrobielle in-vivo-Wirksamkeit (oral) bei Mäuse-Candidose

Mäuse vom Typ SPF-$CF_1$ werden intravenös mit 1-2 x $10^6$ logarithmisch wachsenden Candida-Zellen, suspendiert in physiologischer Kochsalzlösung, infiziert. Die Tiere werden mit Dosierungen von 12,5 bis 25mg/kg Körpergewicht 2 x täglich über 5 Tage oral behandelt.

Unbehandelte Tiere starben 3 bis 6 Tage post infektionem. Die Ueberlebensrate am 6.Tag post infektionem betrug bei unbehandelten Kontrolltieren etwa 5 %.

In diesen Versuchen zeigten die erfindungsgemäßen Formen B bessere Wirksamkeit als die bekannten geometrischen Isomerengemische.

Die Ergebnisse sind in Tabelle B zusammengefaßt.

Tabelle B: Antimikrobielle in-vivo-Wirksamkeit (oral) bei Mäuse-Candidose

| Wirkstoff | Wirkung |
|---|---|
| 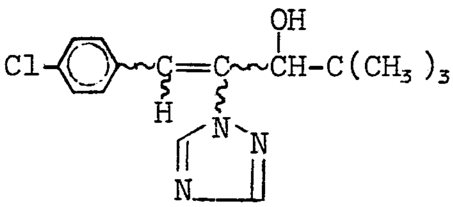 geometrisches Isomerengemisch (bekannt) | +++ |
| Form A | kW |
| Form B(1) | +++++ |

+++++ = sehr gute Wirkung = >95% Ueberlebende am 6.Tag p.i.
++++ = gute Wirkung = ≥80% Ueberlebende am 6.Tag p.i.
+++ = Wirkung = ≥60% Ueberlebende am 6.Tag p.i.
++ = schwache Wirkung = ≥40% Ueberlebende am 6.Tag p.i.
+ = Spur Wirkung = <40% Ueberlebende am 6.Tag p.i.
kW = keine Wirkung

Beispiel C

Antimikrobielle in-vivo-Wirksamkeit (lokal und oral) am Modell der experimentellen Meerschweinchen-Trichophytie

Versuchsbeschreibung:

Weiße Meerschweinchen der Rasse Pirbright-white werden auf dem geschorenen, nicht skarifizierten Rücken mit einer Mikro- und Makrokonidien-Suspension von Trichophyton mentagrophytes infiziert. Bei unbehandelten Tieren entwickelt sich innerhalb 12 Tagen p.i. das typische Bild einer Dermatophytose mit Rötung, Schuppung und Haarausfall bis zum totalen Integument-Defekt an der Infektionsstelle. Die infizierten Tiere werden

a) beginnend mit dem 3.Tag p.i. 1x täglich mit 1%-iger Lösung der erfindungsgemäßen Präparate (in Dimethylsulfoxid : Glycerin = 1 : 4), lokal behandelt;

b) beginnend mit dem Tag der Infektion 1 x täglich mit 25 mg/kg Körpergewicht der erfindungsgemäßen Präparate (in wässriger Lösung) oral behandlet.

Die erfindungsgemäßen Formen B zeigten bessere Wirksamkeiten als die bekannten geometrischen Isomerengemische.

Die Ergebnisse sind in Tabelle C zusammengefaßt.

Tabelle C: Antimikrobielle in-vivo-Wirksamkeit (lokal und oral) am Modell der experimentellen Meerschweinchen-Trichophytie

| Wirkstoff | Wirkung lokale Therapie (1%-ige Lösung) | Wirkung orale Therapie (50mg/kg/die) |
|---|---|---|
| 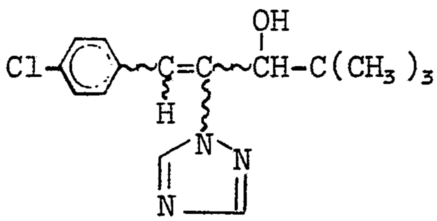 geometrisches Isomerengemisch (bekannt) | schwache Wirkung | — |
| Form A | — | — |
| Form B | sehr gute Wirkung | sehr gute Wirkung |

## Herstellungsbeispiele

### Beispiel 1

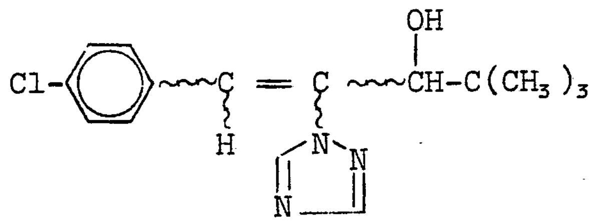

43,5g (0,15 Mol) der geometrischen Form B von 1-(4-Chlorphenyl)-4,4-dimethyl-2-(1,2,4-triazol-1-yl)-1-penten-3-on werden in 500 ml Isopropanol suspendiert und portionsweise mit 2,85 g (0,075 Mol) Natriumborhydrid versetzt. Man läßt das Reaktionsgemisch 21 Stunden bei Raumtemperatur rühren und engt anschließend im Vakuum ein. Der Rückstand wird mit verdünnter Essigsäure behandelt und mit Ether extrahiert. Die vereinigten Etherphasen werden mit Wasser gewaschen, über Natriumsulfat getrocknet und eingeengt. Das resultierenden Oel wird durch Verrühren mit Diisopropylether zur Kristallisation gebracht. Nach Umkristallisation aus Acetonitril erhält man 10,5g (25 % der Theorie) der geometrischen Form B von 1-(4-Chlorphenyl)-4,4-dimethyl-2-(1,2,4-triazol-1-yl)-1-penten-3-ol vom Schmelzpunkt 160°C.

(Reduktion in Gegenwart von Cer-III-chlorid)
Zu 37,3g (0,1 Mol) Cer-III-chlorid-heptahydrat in 250 ml Methanol werden 29g (0,1 Mol) der geometrischen Form B von 1-(4-Chlorphenyl)-4,4-dimethyl-2-(1,2,4-triazol-1-yl)-1-penten-3-on gegeben. Aus der anfänglich klaren Lösung scheiden sich bald Kristalle ab. Nach portionsweiser Zugabe von 3,8g (0,1 Mol) Natriumborhydrid wird das Reaktionsgemisch 5 Stunden auf 50°C erhitzt. Man läßt abkühlen, verdünnt mit Wasser und extrahiert mit Ether. Die Etherphase wird über Natriumsulfat getrocknet und eingeengt. Die zurückbleibende

Kristallmasse wird aus Acetonitril umkristallisiert. Man erhält man 13,5g (46 % der Theorie) der geometrischen Form B von 1-(4-Chlorphenyl)-4,4-dimethyl-2-(1,2,4-triazol-1-yl)-1-penten-3-ol vom Schmelzpunkt 160°C.

Herstellung des Ausgangsproduktes

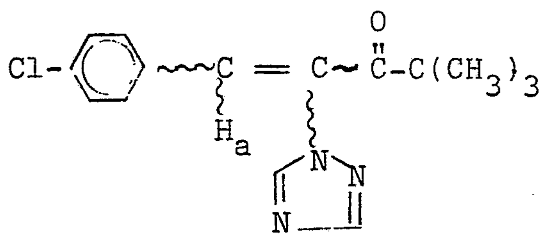

167g (1 Mol) 3,3-Dimethyl-1-(1,2,4-triazol-1-yl)-butan-2-on und 140,5g (1 Mol) 4-Chlorbenzaldehyd werden in 700 ml Toluol gelöst und mit 1,2g (0,02 Mol) Eisessig und 1,8 ml (0,02 Mol)Piperidin versetzt. Das Reaktionsgemisch wird 40 Stunden auf 120°C erhitzt, wobei das Reaktionswasser laufend abgetrennt wird. Danach wird das Reaktionsgemisch im Vakuum eingeengt und das zurückbleibende Oel destilliert (Siedebereich 130-160°C/0,04 Torr). Das Destillat wird mit Ethanol verrührt, wobei sich Kristalle abscheiden. Diese werden abgesaugt und getrocknet. Man erhält so 35g (12 % der Theorie) der geometrischen Form B von 1-(4-Chlorphenyl)-44 -dimethyl-2-(1,2,4-triazol-1-yl)-1-penten-3-on vom Schmelzpunkt 110°C ($\delta$ $H_a$ = 7,1 ppm in $CDCl_3$).
Die Ethanol-Mutterlauge wird eingeengt und das zurückbleibende Oel in Diisopropylether gelöst, wobei es zur Auskristallisation kommt. Nach Umkristallisieren aus Diisopropylether erhält man 25,5g (8,8 % der Theorie) der geometrischen Form A von 1-(4-Chlorphenyl)-4,4-dimethyl-2-(1,2,4-triazol-1-yl)-1-penten-3-on vom Schmelzpunkt 82°C ($\delta$ $H_a$ = 7,5 ppm in $CDCl_3$).

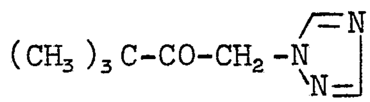

138g (2 Mol) 1,2,4-Triazol werden bei Raumtemperatur portionsweise zu 276,4g (2 Mol) gemahlenem Kaliumcarbonat und 269,2g (2 Mol) α-Chlorpinakolin in 500 ml Aceton gegeben, wobei die Innentemperatur bis zur Siedehitze ansteigt. Man läßt 5 Stunden unter Rückfluß rühren und kühlt dann auf Raumtemperatur ab. Das Reaktionsgemisch wird filtriert und das Filtrat durch Abdestillieren des Lösungsmittels im Vakuum eingeengt. Der ölige Rückstand kristallisiert nach Zugabe von Benzin. Man erhält 240,8g (72 % der Theorie) 3,3-Dimethyl-1-(1,2,4-triazol-1-yl)-butan-2-on vom Schmelzpunkt 62-64°C.

In entsprechender Weise werden die nachfolgenden Beispiele der allgemeinen Formel

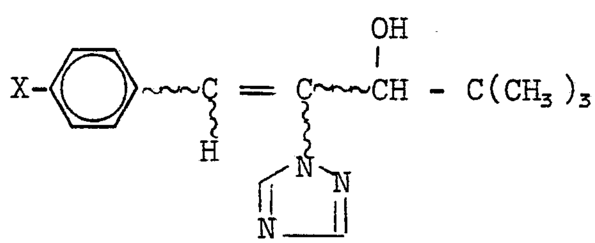 (I)

erhalten:

| Bsp.Nr. | X | Schmelzpunkt(°C) |
|---|---|---|
| 2 | F | (Form B) |
| 3 | 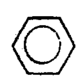 | (Form B) |
| 4 | 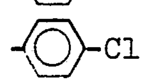 | (Form B) |

Patentansprüche

1. Geometrische Formen B von 4,4-Dimethyl-1-phenyl-2-(1,2,4-triazol-1-yl)-1-penten-3-olen der allgemeinen Formel

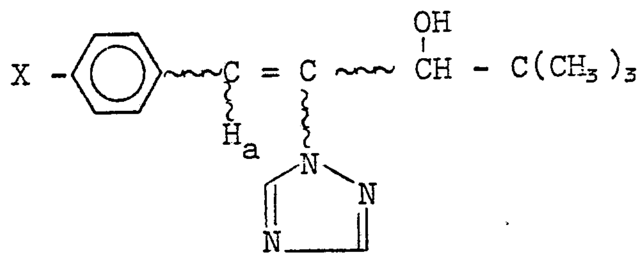

(I)

in welcher

X für Halogen, Cynao oder gegebenenfalls substituiertes Phenyl steht,

und deren physiologisch verträglichen Säureadditions-Salze.

2. Geometrische Formen der allgemeinen Formel (I) in Anspruch 1,

in welcher

X für Fluor, Chlor, Cyano oder gegebenenfalls durch Chlor substituiertes Phenyl steht.

3. Geometrische Formen der allgemeinen Formel (I) in Anspruch 1, in welcher X für Chlor steht.

4. Verfahren zur Herstellung der geometrischen Formen der allgemeinen Formel (I) in Anspruch 1, dadurch gekennzeichnet, daß man die geometrischen Formen B von 4,4-Dimethyl-1-phenyl-2-(1,2,4-triazol-1-yl)-1-penten-3-onen der allgemeinen Formel

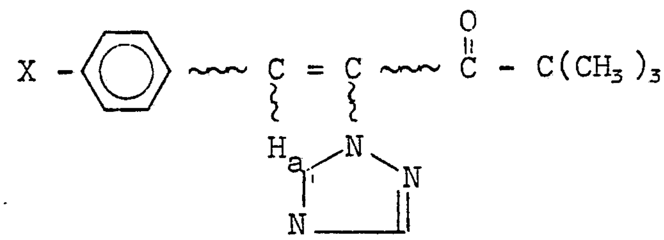 (II)

in welcher

X die oben angegebene Bedeutung hat,

entweder mit komplexen Hydriden, gegebenenfalls in Gegenwart eines Verdünnungsmittels,

oder mit Aluminium-isopropylat in Gegenwart eines Verdünnungsmittels reduziert.

5. Arzneimittel, gekennzeichnet durch einen Gehalt an mindestens einer geometrischen Form gemäß Anspruch 1.

6. Verfahren zur Herstellung von antimykotischen Mitteln, dadurch gekennzeichnet, daß man geometrische Formen gemäß Anspruch 1 mit inerten, nichttoxischen pharmazeutisch geeigneten Trägerstoffen vermischt.

7. Verfahren zur Behandlung von Mykosen, dadurch gekennzeichnet, daß man geometrische Formen gemäß Anspruch 1 Menschen oder Tieren appliziert, die an Mykosen erkrankt sind.

0020955

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung

EP 80102452.2

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int. Cl.³) |
|---|---|---|---|
| D | DE - A1 - 2 838 847 (SUMITOMO) (15-03-1979)<br>+ Patentansprüche; Seiten 7-10, 13,14,17 +<br>-- | 1-4 | C 07 D 249/08<br>A 61 K 31/41 |
| | DE - A1 - 2 738 725 (BASF) (08-03-1979)<br>+ Patentansprüche; Seiten 5,6 +<br>-- | 1-4 | |
| | DE - A1 - 2 713 777 (BAYER)<br>+ Patentansprüche +<br>-- | 1-4 | **RECHERCHIERTE SACHGEBIETE (Int. Cl.³)** |
| | US - A - 4 104 399 (POMMER)<br>+ Gesamt +<br>---- | 1-4 | C 07 D 249/00<br>A 61 K 31/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6

Unvollständig recherchierte Patentansprüche: -

Nicht recherchierte Patentansprüche: 7

Grund für die Beschränkung der Recherche:

Verfahren zur therapeutischen Behandlung des menschlichen oder tierischen Körpers, (Art. 52(4) EPÜ)

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| Recherchenort | Abschlußdatum der Recherche | Prüfer |
|---|---|---|
| WIEN | 23-07-1980 | HAMMER |